(19) Europäisches Patentamt
European Patent Office
Office européen des brevets

(11) Veröffentlichungsnummer: **0 101 979**
**B1**

(12) **EUROPÄISCHE PATENTSCHRIFT**

(45) Veröffentlichungstag der Patentschrift: **30.03.88**

(51) Int. Cl.⁴: **C 12 Q 1/26,** G 01 N 33/52

(21) Anmeldenummer: **83107699.7**

(22) Anmeldetag: **03.08.83**

# (54) Diagnostische Vorrichtung und ihre Verwendung.

(30) Priorität: **23.08.82 DE 3231288**

(43) Veröffentlichungstag der Anmeldung: **07.03.84 Patentblatt 84/10**

(45) Bekanntmachung des Hinweises auf die Patenterteilung: **30.03.88 Patentblatt 88/13**

(84) Benannte Vertragsstaaten: **AT BE CH FR GB IT LI LU NL SE**

(56) Entgegenhaltungen:
**GB - A - 893 301**
**US - A - 3 867 259**
**US - A - 4 266 022**

(73) Patentinhaber: **Medi-Pharma Vertriebsgesellschaft mbH, Sachsenring 37-47, D-5000 Köln 1 (DE)**

(72) Erfinder: **Steinbach, Roland Wilfried, Lothringerstrasse 14, D-5000 Köln 1 (DE)**
Erfinder: **Roy, Asok Kumar, Dr., Strünckweg 11, D-1000 Berlin 13 (DE)**
Erfinder: **Krauss, Peter, Dr., Asternweg 211, D-5024 Pulheim (DE)**

(74) Vertreter: **Groening, Hans Wilhelm, Dipl.-Ing., Patentanwälte Strehl Schübel-Hopf Groening Schulz Widenmayerstrasse 17 Postfach 22 03 45, D-8000 München 22 (DE)**

**EP 0 101 979 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine diagnostische Vorrichtung zum Nachweis einer erhöhten Konzentration von Dehydrogenasen und/oder Oxidasen und die Verwendung der Vorrichtung.

Für jede zytosolische Dehydrogenase, die im Falle einer pathologischen Veränderung bei Mensch, Tier oder Pflanze in extrazelluläre Flüssigkeiten übergeht, kann in diesen Flüssigkeiten der pathologische Schwellenwert empirisch ermittelt werden. Bei der Prüfung auf eine pathologische Veränderung sollen die physikalisch-chemischen Parameter des Diagnosesystems so eingestellt sein, dass der pathologische Schwellenwert der Dehydrogenase- bzw. Oxidase-Aktivität unter den Diagnosebedingungen mit dem Umschlagspunkt eines Indikators, z.B. eines Redox-Farbstoffs, zusammenfällt. Bei pathologischen Veränderungen der Eigenschaften von Zellmembranen ist zu erwarten, dass verschiedene Pyridin-abhängige Dehydrogenasen, wie Lactatdehydrogenase (LDH), Alkoholdehydrogenase (ADH), Glycerinaldehyd-3-phosphatdehydrogenase, Isocitratdehydrogenase, Glutamatdehydrogenase, Glucosedehydrogenase und Glucose-6-phosphatdehydrogenase, und/oder Flavin-abhängige Dehydrogenasen und/oder Flavin-abhängige Oxidase, wie Succinat-Dehydrogenase, Glycerin-3-phosphatdehydrogenase, Glucose-Oxidase ins Serum und in andere extrazelluläre Flüssigkeiten übergehen und für jede Dehydrogenase bzw. Oxidase jeweils ein für die Diagnose pathologischer Schwellenwert gefunden wird.

Als pathologische Veränderung kommt hier z.B. die Bildung vom Malignomen in Betracht. Zur Anpassung des Umschlagspunktes eines Redox-Farbstoffs an den jeweiligen pathologischen Schwellenwert können die physikalisch-chemischen Parameter des Diagnosesystems z.B. in folgender Weise eingestellt werden:

a. Es wird das Substrat der jeweiligen Dehydrogenase eingesetzt, z.B. Lactat für LDH.

b. Die Mengen des vorgelegten Substrats und des gleichzeitig eingesetzten Pyridinnucleotids werden eingestellt.

c. Der pH-Wert und damit die Lage des Reaktionsgleichgewichts wird eingestellt, gegebenenfalls mittels eines Puffersystems.

d. Es wird ein Redox-Farbstoff (z.B. Leukofarbstoff, der von farblos nach farbig umschlägt) gewählt, dessen Redox-Potential unter den Diagnosebedingungen über dem Redox-Potential des Pyridinnucelotid-Systems liegt.

e. Es wird ein Elektronenüberträger gewählt, dessen Redox-Potential zwischen dem des Pyridinnucleotid-Systems und dem des gewählten Redox-Farbstoffs liegt.

f. Solange das Gleichgewicht der Dehydrogenase-Reaktion noch nicht eingestellt ist, ergibt eine längere Umsetzungszeit eine höhere Konzentration des reduzierten Pyridinnucleotids. Dementsprechend kann die Umschlagsschwelle des Redox-Farbstoffs und damit die Nachweisempfindlichkeit des Diagnosesystems über die Umsetzungsdauer eingestellt werden.

g. Es können unterschiedliche Träger für das Diagnosesystem gewählt werden.

Aus der DE-PS 2 443 741 ist ein Verfahren zur gemeinsamen Bestimmung der Isozyme 4 und 5 der LDH bekannt, bei dem ein Gemisch aus einem Lactat und Nicotinamid-adenin-dinucleotid (NAD) mit der zu untersuchenden Körperflüssigkeit in Berührung gebracht wird. Wenn LDH in erhöhter Menge vorliegt, katalysiert sie die Umsetzung des Lactats mit dem Dinucleotid zum entsprechenden Pyruvat und dem reduzierten Nicotinamid-adenin-dinucleotid (NADH) gemäss folgender Formelgleichung:

$$\text{Lactat} + \text{NAD}^{\oplus} \underset{}{\overset{\text{LDH}}{\rightleftharpoons}} \text{Pyruvat} + \text{NADH} + \text{H}^{\oplus}$$

Die LDH tritt in menschlichen Zellen in Form der Isoenzyme 1, 2, 3, 4 und 5 auf. Bei bestimmten spezifischen pathologischen Veränderungen treten erhöhte Werte der LDH auf, z.B. kommt es bei Malignomen im weiblichen Genitalbereich zu einer Erhöhung der Isoenzyme 4 und 5.

Gemäss der Druckschrift muss die Umsetzung zwischen dem Lactat und dem NAD bei einem pH-Wert von 6 bis 6,5 durchgeführt werden. Zu diesem Zweck werden die Körperflüssigkeit und/oder das Lactat und das NAD enthaltende Testreagens auf diesen pH-Bereich, gegebenenfalls unter Verwendung eines Puffersystems, eingestellt. In Fällen, in denen eine Körperflüssigkeit, z.B. Vaginalsekret, einen niedrigeren pH-Wert, z.B. den pH-Wert 4, aufweist, kann das Testreagens auf einen pH-Wert eingestellt werden, der den Wert 6,5 überschreitet, damit sich bei der Testreaktion der gewünschte pH-Wert im Bereich von 6 bis 6,5 ergibt. Die in der vorstehenden Formelgleichung nach rechts laufende Umsetzung wird durch eine Folgereaktion sichtbar gemacht. Zu diesem Zweck wird das Testreagens noch mit Nitroblautetrazoliumchlorid (NBT) und gegebenenfalls Phenazinmethosulfat (N-Methylphenazoniummethylsulfat, 5-Methylphenazoniummethylsulfat, PMS) als Wasserstoffüberträger bzw. Elektronenüberträger versetzt. Das gemäss der Formelgleichung gebildete NADH wird in Gegenwart des Wasserstoffüberträgers bzw. Elektronenüberträgers von dem NBT zu NAD oxidiert, wobei das NBT in den blauen Farbstoff

Nitroblauformazan [4,4′-(3,3′-Dimethoxy-biphenylenyl)-bis-2N-(2N′-nitrophenyl-C-phenylformazan)]

übergeht und so die enzymatische Aktivität und Anwesenheit der LDH bzw. ihrer Isoenzyme sichtbar werden lässt.

Das bekannte Verfahren wurde auf die Untersuchung des Vaginalsekrets von Frauen angewandt. Die Zeitdauer der Umsetzung zwischen diesem Sekret und dem Diagnosereagens betrug vorzugsweise 15 bis 30 Minuten. Es wurden pathologische Veränderungen im weiblichen Genitaltrakt, z.B.

Carcinoma colli uteri, Carcinoma corporis und Carcinoma in situ festgestellt.

Bei der Vorsorgeuntersuchung von z.B. Frauen auf Genitalkarzinome nach anderen bekannten Verfahren, z.B. der Zytologie und der Kolposkopie, ergeben sich für Collum- und Cervixkarzinome falsch-negative Diagnosen in der Grössenordnung von durchschnittlich 30% der Befunde. Dabei werden andere als die oben angeführten Genitalkarzinome der Frau gar nicht miterfasst. Es besteht deshalb ein grosses Bedürfnis dafür, diese Fehlerquote zu senken.

In der GB-PS 893 301 ist ein kolorimetrischer Test für Serum-Enzyme beschrieben. Zur Bestimmung von Lactatdehydrogenase in biologischen Flüssigkeiten wird dort ein Gemisch angegeben, das ein Salz der Milchsäure, Diphosphopyridinnucleotid (Nicotinamid-adenin-dinucleotid), Diaphorase und 2,6-Dichlorindophenol enthält. Der pH-Wert des Gemisches soll im Bereich von 5 bis 9 liegen.

Jedoch wurde gemäss dieser Druckschrift ein pH-Wert von unter 7,0 offensichtlich nicht ernsthaft in Betracht gezogen. Denn der als bevorzugt angegebene Bereich liegt bei pH 7 bis 8, und ein spezielles Ausführungsbeispiel wird bei einem pH-Wert von 7,4 durchgeführt. An einer anderen Textstelle ist angegeben, dass der Test in einer mit einem Puffer vom pH-Wert 7 sorgfältig gepufferten Lösung erfolgt. Schliesslich hat sich gezeigt, dass dieser Test bei einem pH-Wert von 6 tatsächlich nicht durchführbar ist. Der Einsatz von Tetrazoliumsalzen in Zusammenhang mit der Bestimmung von LDH ist in der Druckschrift nicht erwähnt.

Im übrigen ist dieser bekannte Test ganz allgemein zur Bestimmung von Serum-Enzymen, nicht aber zum Nachweis von ausschliesslich pathologisch erhöhten Konzentrationen von Dehydrogenasen vorgesehen. Dementsprechend soll der bekannte Test bereits bei sehr geringen Enzym-Konzentrationen ansprechen, so dass eine Differenzierung normaler und pathologisch erhöhter Dehydrogenase- bzw. Oxidase-Werte nicht möglich ist.

Der Erfindung liegt die Aufgabe zugrunde, eine diagnostische Vorrichtung zum Nachweis einer pathologisch erhöhten Konzentration von Dehydrogenasen in Flüssigkeiten oder Sekreten von Menschen, Tieren oder Pflanzen anzugeben. Die Vorrichtung soll eine hohe diagnostische Treffsicherheit gewährleisten und sich insbesondere zum Erkennen von Malignomen eignen. Ausserdem soll die Diagnose in möglichst kurzer Zeit durchführbar sein.

Diese Aufgabe wird erfindungsgemäss durch eine diagnostische Vorrichtung gelöst, die aus einem Träger, der einen Redox-Farbstoff bindet, sowie einem auf einen pH-Wert im sauren Bereich eingestellten Stoffgemisch aus dem der jeweiligen Dehydrogenase entsprechenden Substrat, einer wasserstoffübertragenden Verbindung und mindestens einem Redox-Farbstoff besteht. Die Vorrichtung ist dadurch gekennzeichnet, dass ein aus Cellulosefasern gebildeter, polare Gruppen aufweisender Träger verwendet wird und das Stoffgemisch zur Erfassung von ausschliesslich pathologisch erhöhten Konzentrationen der jeweiligen Dehydrogenase und/oder Oxidase auf einen pH-Wert von unter 5,0 eingestellt ist.

Die diagnostische Vorrichtung eignet sich besonders gut zur Erkennung von pathologischen Veränderungen, die von einer Erhöhung der Konzentration von Dehydrogenasen in extrazellulären Flüssigkeiten begleitet sind. Solche Konzentrationsänderungen treten auch schon in Frühstadien von Malignomen auf, in denen die Aussicht auf eine erfolgreiche Therapie noch besonders gross ist. Die diagnostische Vorrichtung gibt einen Hinweis auf Malignome an sehr unterschiedlichen Organen. So können unter anderem Karzinome an typisch weiblichen Organen, wie im weiblichen Genitalbereich, festgestellt werden. Auch sind Infektionen durch Bakterien, Parasiten und Pilze mittels der Vorrichtung nachweisbar.

Ein besonderer Vorteil der diagnositischen Vorrichtung ist ihr hoher Prognosewert der Diagnosen. Beispielsweise wurde bei Reihenversuchen an Frauen mit histologisch gesicherten Karzinomen im Genitalbereich für die Stadien I bis IV gefunden, dass nur weniger als 10% aller Diagnosen falsch-negativ sind.

Im Zusammenhang mit dieser Farbstoffbildung haben spezielle Untersuchungen, in die z.B. auch Gewebeschnitte einbezogen worden sind, ergeben, dass der Farbstoff während und nach seiner Bildung überraschenderweise auf dem Trägermaterial bleibt und deshalb keine schädlichen Sekundärreaktionen im und am Körper des untersuchten Patienten auslöst.

Diese Vorteile machen die diagnostische Vorrichtung besonders wertvoll für gesundheitspolitisch wünschenswerte Vorsorgeuntersuchungen, bei denen nicht nur ein möglichst hohes Mass an diagnostischer Sicherheit, sondern auch eine grosse Sicherheit vor schädlichen Sekundärreaktionen des Diagnoseverfahrens bei gleichzeitig möglichst einfacher Methodik gegeben sein muss.

Die hervorragenden Eigenschaften der diagnostischen Vorrichtung sind auf die besondere Kombination aus einem Träger mit polaren Gruppen und einem niedrigen pH-Wert des Stoffgemisches auf dem Träger zurückzuführen. Es ist überraschend, dass gerade dadurch eine Differentialdiagnostik möglich ist, die mit hoher Treffsicherheit die pathologischen Fälle erfasst.

Die grosse Bedeutung des Trägers für die Funktion der diagnostischen Vorrichtung zeigt sich z.B. darin, dass ohne Verwendung des Trägers die Farbreaktion des Stoffgemisches mit einer eine pathologisch erhöhte Dehydrogenase-Konzentration enthaltenden Flüssigkeit im genannten niedrigen pH-Bereich nicht abläuft.

Die zur Diagnose herangezogene Flüssigkeit kann aus sehr unterschiedlichen Bereichen bei Mensch, Tier oder Pflanze stammen. Beispielsweise kann zur Prüfung auf bösartige Neoplasmen im Bereich der Genitalorgane von Frauen das Vaginalsekret dienen. Die diagnostische Vorrichtung ist aber auch z.B. auf Blutserum, Magen- und

Darmsaft, Gallenflüssigkeit, Bronchial-, Prostata- und Urethalsekret sowie Sputum anwendbar.

Falls es sich bei der nachzuweisenden Dehydrogenase um LDH handelt, wird vorzugsweise Natriumlactat eingesetzt. Es können aber auch andere entsprechende Verbindungen, z.B. Kaliumlactat oder Calciumlactat, verwendet werden.

Die wasserstoffübertragende Verbindung ist ein Stoff, der die Reduktion des Redox-Farbstoffs, z.B. eines Tetrazoliumsalzes, unterstützt. Beispiele für derartige wasserstoffübertragende Verbindungen sind NAD, Nicotinamid-adenin-dinucleotidphosphat (NADP), Flavin-adenin-dinucleotid (FAD) und Flavinmononucleotid (FMN). Es können aber auch andere, dem Fachmann geläufige Verbindungen eingesetzt werden, die sich z.B. bei der durch die vorstehende Formelgleichung erläuterten Umwandlung von Lactat in Pyruvat anstelle des NAD in eine dem NADH entsprechende reduzierte Form überführen lassen.

Um in einer zu untersuchenden Flüssigkeit eine Dehydrogenase-Konzentration sichtbar zu machen, die über dem pathologischen Schwellenwert liegt, ist mindestens ein Redox-Indikator, vorzugsweise ein Redox-Farbstoff, in dem Stoffgemisch auf dem Träger enthalten.

Als Farbstoffkomponente kommen z.B. Tetrazoliumsalze, Benzidinfarbstoffe, Indophenole, wie 2,6-Dichlorindophenol oder Dibromindophenol, oder ABTS in Betracht. Entsprechende spezielle Tetrazoliumsalze sind nachfolgend angegeben:

NBT
TNBT
MTT
INT
BT
TV
TT
NT
Trinitro-TT
Dinitro-TT
Tetranitro-NT
Tetranitro-BT
Nitro-BT-methyl (Methoxygruppe substituiert durch Methylgruppe)
Dinitro-TV
3-(4-Biphenyl)-2,5-di-p-nitrophenyl-tetrazoliumchlorid
Nitro-NT
Tetranitro-$BT_{1/2}$ (symmetrisch geteiltes Tetranitro-BT)
Nitro-BT
3-(3,3′-Dimethoxy-4-biphenylylen)-2-p-nitrophenyl-5-phenyltetrazoliumchlorid
Nitro-TV
Nitro-TT
3-p-Jodophenyl-5-nitrophenyl-2-phenyl-tetrazoliumchlorid
5,5′-Dinitro-NT
5,5′-Dinitro-BT
5-Nitro-TV
5-Nitro-TT
2-(3-Methoxy-4-phenyl)-5-p-nitrophenyl-3-phenyltetrazoliumchlorid

Dabei bedeuten:

NBT = 3,3′-(3,3′-Dimethoxy-4,4′-biphenylen)-bis-(2-p-nitrophenyl-5-phenyl-2H-tetrazoliumchlorid)
TNBT = 3,3′-(3,3′-Dimethoxy-4,4′-bisphe-nylen)-bis-(2,5-p-nitrophenyl-2H-tetra-zoliumchlorid)
MTT = 3-(4,5-Dimethyl-2-thiazolyl)-2,5-diphenyl-2H-tetrazoliumbromid
INT = 2-p-Jodphenyl-3-p-nitrophenyl-5-phenyl-2H-tetrazoliumchlorid
BT = 3,3′-(3,3′-Dimethoxy-4,4′-biphenylen)-bis-(2,5-diphenyl-2H-tetrazoliumchlorid)
TV = 3α-Naphthyl-2,5-diphenyl-2H-tetrazoliumchlorid
TT = 2,3,5-Triphenyl-2H-tetrazoliumchlorid
NT = 3,3′-(4,4′-biphenylen)-bis-(2,5-diphenyl-2H-tetrazoliumchlorid
ABTS = 2,2′-Azino-bis[3,3′-äthyl-benzthiazolin-6,6′-sulfonsäure]

Mit den vorstehenden Tetrazoliumsalzen kann der Farbumschlag der diagnostischen Vorrichtung besonders gut auf den pathologischen Schwellenwert der nachzuweisenden Dehydrogenasen eingestellt werden. Deshalb sind Tetrazoliumsalze, insbesondere NBT, als Redox-Farbstoffe bevorzugt.

Das Stoffgemisch auf dem Träger der diagnostischen Vorrichtung weist eine pH-Wert von unter 5,0, vorzugsweise 3,0 bis unter 5,0, insbesondere 4,5, auf. Es ist überraschend, dass die Reaktion, die bei der Verwendung der Vorrichtung z.B. durch die LDH katalysiert wird, in einem derartig niedrigen pH-Bereich noch abläuft, da, wie aus der vorstehenden Formelgleichung ersichtlich ist, bei der Umsetzung des Lactats zum Pyruvat Wasserstoffionen frei werden. Man hätte deshalb erwarten müssen, dass bei einer weiteren Erhöhung der Wasserstoffionenkonzentration über einen pH-Wert von 6,0 hinaus, wie er aus der DE-PS 2 443 741 bekannt ist, die genannte Reaktion noch langsamer oder nicht mehr abläuft bzw. das in der Formelgleichung dargestellte Gleichgewicht nach links verschoben wird. Entgegen diesen Erwartungen aber verläuft die Reaktion ohne ersichtliche Verlangsamung in der gewünschten Weise, wobei auch noch, wie erwähnt, die diagnostische Treffsicherheit der erfindungsgemässen Vorrichtung gegenüber bekannten Verfahren verbessert wird.

Die Einstellung des gewünschten pH-Werts des Stoffgemisches kann in üblicher Weise, z.B. durch Zugabe von Salzsäure und/oder Phosphorsäure, erfolgen.

Gemäss einer bevorzugten Ausführungsform der diagnostischen Vorrichtung enthält das Stoffgemisch zur Einstellung und/oder Aufrechterhaltung des pH-Wertes ein Puffersystem. Dadurch wird bei der Verwendung der diagnostischen Vorrichtung das Reaktionsmedium in einem günstigen pH-Bereich gehalten, auch wenn die untersuchte Flüssigkeit einen davon abweichenden pH-Wert aufweist.

Vorzugsweise liegt das Puffersystem in Form von Triäthanolaminhydrochlorid oder in Form eines nicht äquivalenten Gemisches aus Triäthanolamin und Chlorwasserstoff vor. Der Chlorwasserstoff wird z.B. in Form von Salzsäure eingesetzt.

Es können aber auch andere Puffersysteme, z.B. ein Phosphatpuffer, benutzt werden, soweit sie nicht zu unerwünschten Nebenreaktionen führen. Geeignete Puffersysteme sind dem Fachmann bekannt.

Gemäss einer vorteilhaften Weiterbildung der diagnostischen Vorrichtung enthält das Stoffgemisch zusätzlich eine Verbindung, die als Wasserstoffüberträger oder Elektronenüberträger wirkt. Diese Verbindung katalysiert die Reduktion z.B. eines Tetrazoliumsalzes zum entsprechenden farbigen Formazan. Dazu muss der Wasserstoff- oder Elektronenüberträger ein Redox-Potential aufweisen, das zwischen dem Redox-Potential des erfindungsgemäss eingesetzten Redox-Systems (z.B. des NAD/NADH-Systems) und dem Redox-Potential des Redox-Farbstoffes (z.B. des Tetrazoliumsalzes) liegt.

Spezielle Beispiele für derartig wirkende Verbindungen sind Phenazinmethosulfat (PMS), Diaphorasen, Meldola-Blau und Menadion. Andere entsprechende Verbindungen sind dem Fachmann bekannt.

Das nachfolgende Formelschema erläutert beispielshaft die bei der Anwendung der diagnostischen Vorrichtung ablaufenden Reaktionen, wenn das Redox-System NAD/NADH, ferner PMS als Elektronenüberträger und das Tetrazoliumsalz NBT eingesetzt werden:

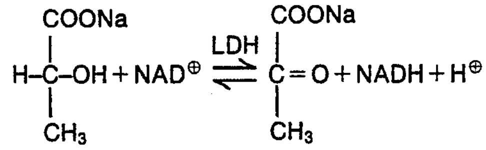


Lactat Pyruvat

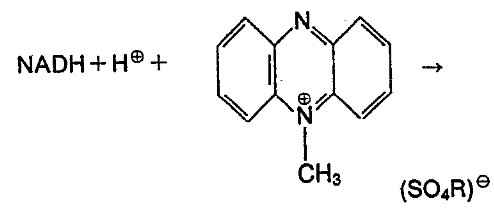

Phenazinmethosulfat

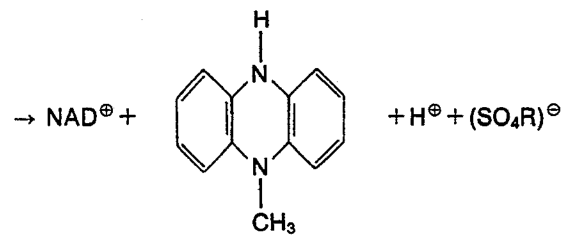

N-Methyldihydrophenazin

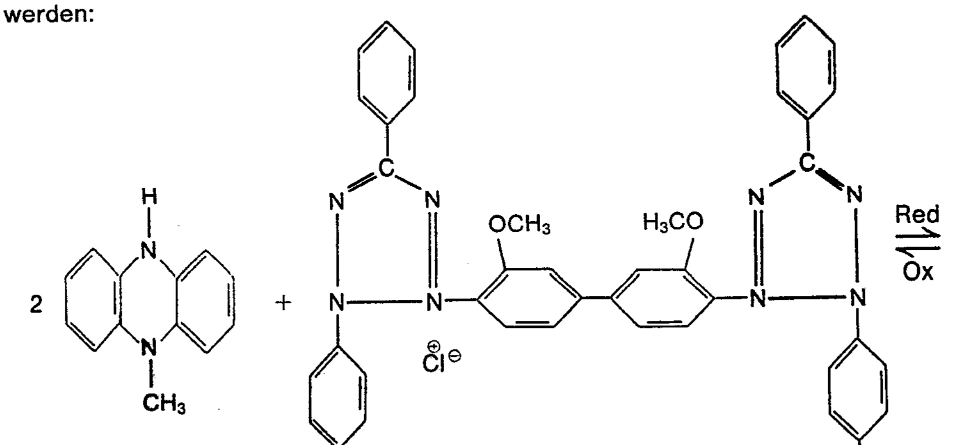


N-Methyldihydrophenazin NBT

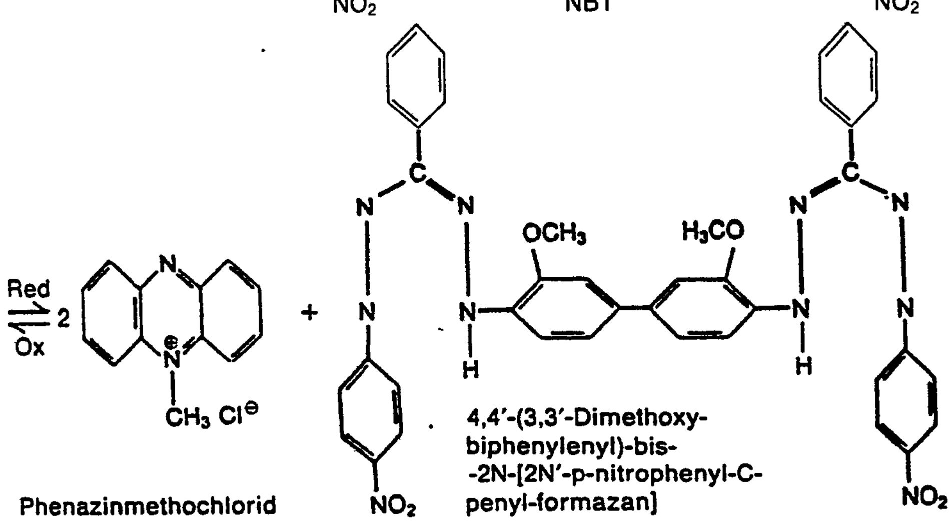


Phenazinmethochlorid

4,4'-(3,3'-Dimethoxy-biphenylenyl)-bis--2N-[2N'-p-nitrophenyl-C-penyl-formazan]

Für diagnostische Vorrichtungen, die zur Feststellung von Karzinomen im weiblichen Genitalbereich vorgesehen sind, haben sich Träger in Form von Tampons aus Cellulosefasern besonders bewährt. Die Tampons werden bei ihrer Verwendung in die Vagina eingeführt und ermöglichen so eine Reaktion des Stoffgemisches auf dem Tampon mit dem Vaginalsekret. Es ist jedoch auch eine extrakorporale Durchführung des Tests möglich, indem man z.N. entnommenes Vaginalsekret ausserhalb des Körpers mit dem imprägnierten Tampon in Kontakt bringt.

Grundsätzlich kommen aber auch andere Trägerformen, wie Stäbchen oder Folien, in Betracht.

Das Material des Trägers kann eine faserige, körnige oder andersartige Struktur aufweisen. In jedem Fall weist der Träger polare Gruppen auf.

Die Erfindung bezieht sich auch auf die Verwendung der diagnostischen Vorrichtung zur frühzeitigen Erkennung von pathologischen Veränderungen, insbesondere malignen Erkrankungen, an Menschen, Tieren und Pflanzen.

Eine bevorzugte Verwendung der diagnostischen Vorrichtung ist ihre Verwendung zum Nachweis einer pathologisch erhöhten Konzentration von Dehydrogenasen in menschlichen, tierischen oder pflanzlichen extrazellulären Flüssigkeiten, wobei das Stoffgemisch auf dem Träger mit der zu untersuchenden Flüssigkeit umgesetzt wird, mit der Massgabe, dass die Umsetzung des Stoffgemisches mit der zu untersuchenden Flüssigkeit während höchstens 10 Minuten durchgeführt wird. Optimale Ergebnisse werden erhalten, wenn diese Zeit 5 bis 7 Minuten beträgt.

Dies gilt insbesondere für den Fall, dass der Träger der diagnostischen Vorrichtung ein Tampon ist, der zur Diagnose in die Vagina einer zu untersuchenden Frau eingeführt wird. Die Liegezeit des Tampons in der Vagina entspricht dann den vorgenannten Zeiten. Beträgt die Liegezeit mehr als 10 Minuten, kann in Einzelfällen eine Farbreaktion an dem Tampon auftreten, auch wenn keine pathologische Veränderung bei der untersuchten Frau vorliegt. Es ist also wesentlich, dass die Liegezeit nicht zu lange gewählt wird.

Die im Rahmen der Erfindung optimale Liegezeit von 5 bis 7 Minuten ist erheblich kürzer als die bevorzugte Liegezeit von 15 bis 30 Minuten gemäss der DE-PS 2 443 741. Dies ist für die Durchführbarkeit in der Praxis besonders wichtig.

Ausserdem hat sich als besonders zweckmässig erwiesen, wenn die Patientin während der Tamponliegezeit umhergeht, um eine intensivere Berührung zwischen dem Tampon und der Scheidenhaut in der Vagina zu bewirken.

Es wurde auch gefunden, dass Flüssigkeiten, die auf eine pathologisch erhöhte Dehydrogenasekonzentration überprüft werden sollen, sehr verschiedene pH-Werte aufweisen können. Beispielsweise wurden bei Frauen mit pathologischen Veränderungen im Bereich der Genitalorgane pH-Werte von 3,9 bis über 7 für das Vaginalsekret gemessen. Überraschenderweise lässt sich die erfindungsgemässe diagnostische Vorrichtung leicht auf solche Reaktionsbedingungen einstellen, ohne an diagnostischer Treffsicherheit zu verlieren. Beispielsweise kann bei einer zu untersuchenden Flüssigkeit mit einem pH-Wert von 5,0 oder höher dieser Wert mittels eines auf einen niedrigeren pH-Wert eingestellten Puffersystems in dem Stoffgemisch, das in der diagnostischen Vorrichtung enthalten ist, für die Umsetzung während der Diagnose in der gewünschten Weise gesenkt werden.

Das nachfolgende Beispiel erläutert die Erfindung.

Beispiel

In einer Reihenuntersuchung in verschiedenen Kliniken wurde die Treffsicherheit der diagnostischen Vorrichtung mit einem Tampon als Träger an 486 Frauen mit diagnostisch gesicherten Krebserkrankungen im Bereich der Genitalorgane überprüft. In der nachfolgenden Tabelle I ist die Verteilung der Diagnosegruppen auf die verschiedenen Altersklassen in absoluten Zahlen und Prozentsätzen angegeben. Die Spalte «O.A.» (= ohne Angabe) bezieht sich auf die Fälle, in denen die untersuchende Klinik zu dieser Altersverteilung keine Angaben gemacht hat.

Tabelle I

Altersverteilungen nach Diagnosegruppen

| Diagnosengruppen | Altersklassen (Jahre)<br><30 | | 30 b.u. 40 | | 40 b.u. 50 | | 50 b.u. 60 | | 60 b.u. 70 | | ≥70 | | O.A. | | Insges. | |
|---|---|---|---|---|---|---|---|---|---|---|---|---|---|---|---|---|
| | abs. | % | abs. | % | abs. | % | abs. | % | abs. | % | abs. | % | abs. | % | abs. | % |
| 1. Mammakarzinom | 1 | 2 | 5 | 8 | 14 | 21 | 12 | 18 | 13 | 20 | 14 | 21 | 7 | 11 | 66 | 100 |
| 2. Ovarialkarzinom | 3 | 4 | 3 | 4 | 9 | 13 | 25 | 35 | 12 | 17 | 8 | 11 | 11 | 15 | 71 | 100 |
| 3. Korpuskarzinom | 0 | 0 | 1 | 2 | 2 | 3 | 16 | 22 | 29 | 40 | 14 | 19 | 10 | 14 | 72 | 100 |
| 4. Kollumkarzinom | 2 | 1 | 27 | 15 | 19 | 10 | 32 | 17 | 43 | 23 | 40 | 22 | 22 | 12 | 185 | 100 |

Tabelle I (Fortsetzung)

Altersverteilungen nach Diagnosegruppen

| Diagnosen-gruppen | Altersklassen (Jahre) <30 | | 30 b.u. 40 | | 40 b.u. 50 | | 50 b.u. 60 | | 60 b.u. 70 | | ≥70 | | O.A. | | Insges. | |
|---|---|---|---|---|---|---|---|---|---|---|---|---|---|---|---|---|
| | abs. | % | abs. | % | abs. | % | abs. | % | abs. | % | abs. | % | abs. | % | abs. | % |
| 5. Vaginal-karzinom | 0 | 0 | 0 | 0 | 2 | 10 | 3 | 15 | 3 | 15 | 9 | 45 | 3 | 15 | 20 | 100 |
| 6. Kollum-karzinom | 0 | 0 | 0 | 0 | 0 | 0 | 1 | 8 | 3 | 25 | 5 | 42 | 3 | 25 | 12 | 100 |
| 7. Andere Malignome | 0 | 0 | 0 | 0 | 2 | 11 | 2 | 11 | 6 | 33 | 6 | 33 | 2 | 11 | 18 | 100 |
| 8. Gutartige Tumoren | 1 | 5 | 2 | 9 | 2 | 9 | 6 | 27 | 4 | 18 | 4 | 18 | 3 | 14 | 22 | 100 |
| 9. Entzündungen | 2 | 17 | 4 | 33 | 1 | 8 | 4 | 33 | 1 | 8 | 0 | 0 | 0 | 0 | 12 | 100 |
| 10. Diagnostische Restgruppe | 0 | 0 | 0 | 0 | 3 | 38 | 1 | 13 | 1 | 13 | 2 | 25 | 1 | 13 | 8 | 100 |
| Insgesamt | 9 | 1,9 | 42 | 8,6 | 54 | 11,1 | 102 | 21,0 | 115 | 23,7 | 102 | 21,0 | 62 | 12,8 | 486 | 100,0 |

O.A. = Ohne Angabe
abs. = absolute Anzahl
% = Prozentsatz, welcher der absoluten Anzahl entspricht

Erläuterungen zu einzelnen diagnostischen Gruppen:

Kollumkarzinom: hierbei sind die diagnostischen Bezeichnungen Kollumkarzinom, Zervixkarzinom und Portiokarzinom zusammengefasst.

Karzinome anderer Lokalisation und Sarkome:
Urethrakarzinom, Mammasarkom, malignes Teratom, Uterussarkom, Lymphosarkom, Fibrosarkom, Psammom, malignes Melanom, Magenkarzinom, Sigmoidkarzinom, Rektumkarzinom, Harnblasenkarzinom, Lungenkarzinom. Die Neoplasmen wurden überwiegend deshalb in die Studie aufgenommen, weil sie invasiv bzw. durch Metastasen den Genitalapparat befallen hatten.

Gutartige Tumoren:
Ovarialfibrom, Ovarial-, Parovarialzyste, Uterus myomatosus, Korpuspolyp, Bauchhöhlenzyste, Leiomyoblastom.

Entzündungen:
Salpingitis, Tuboovarialabszess, Cervicitis, Kolpitis, Vaginitis, Vulvitis, chron. Pyelonephritis.

Diagnostische Restgruppe:
Unterbauchhämatom, Blutung in der Menopause, ohne Angabe einer Diagnose, ohne krankhaften Befund.

Im Rahmen dieser Untersuchung wurden auch die vaginalen pH-Werte der Patientinnen gemessen. In der nachfolgenden Tabelle II sind die Ergebnisse zusammengefasst:

Aus der Tabelle ist ersichtlich, dass sich die Untersuchung auf einen sehr breiten pH-Bereich des Vaginalsekrets erstreckte.

In der nachfolgenden Tabelle III sind die Tamponliegezeiten in Minuten angegeben. Darunter ist jeweils die Zeit zu verstehen, während der ein Diagnosetampon in der Vagina bleibt.

Tabelle II

| PH-Werte o.A | | <3 | | 3 bis <4 | | 4 bis <5 | | 5 bis <6 | | 6 bis <7 | | ≥7 | | Summe | |
|---|---|---|---|---|---|---|---|---|---|---|---|---|---|---|---|
| abs. | % | abs. | % | abs. | % | abs. | % | abs. | % | abs. | % | abs. | % | abs. | % |
| 59 | 12,1 | 2 | 0,4 | 1 | 0,2 | 67 | 13,8 | 124 | 25,5 | 63 | 13,0 | 170 | 35,0 | 486 | 100,0 |

Tabelle III

| Tamponliegezeiten (Min) o.A. abs. | % | 4 abs. | % | 5 abs. | % | 6 abs. | % | 7 abs. | % | ≥8 abs. | % | Insgesamt abs. | % |
|---|---|---|---|---|---|---|---|---|---|---|---|---|---|
| 9 | 1,9 | 8 | 1,6 | 84 | 17,3 | 129 | 26,5 | 239 | 49,2 | 17 | 3,5 | 486 | 100,0 |

In den nachfolgenden Tabellen IV, V, VI, VII, VIII, IX, X sind die Testergebnisse zur Treffsicherheit des diagnostischen Mittels bei den verschiedenen Erkrankungen zusammengefasst.

Erläuterung:

| | | |
|---|---|---|
| 0 | = keine Angabe | |
| (−) | = keine Verfärbung | = Diagnose negativ |
| (+) | = rosa | = Diagnose noch negativ |
| + | = hellblau mit violettem Stich | = Diagnose positiv |
| ++ | = blau | = Diagnose positiv |
| +++ | = dunkelbau | = Diagnose positiv |

Tabelle IV

Testergebnisse bei den Patientinnen mit Mammakarzinom nach Tumorstadien

| Tumorstadien | Testergebnisse 0 abs. | % | (−) abs. | % | (+) abs. | % | + abs. | % | ++ abs. | % | +++ abs. | % | Insgesamt abs. | % |
|---|---|---|---|---|---|---|---|---|---|---|---|---|---|---|
| Stadium I | 0 | 0 | 0 | 0 | 2 | 11 | 6 | 32 | 8 | 42 | 3 | 16 | 19 | 100 |
| Stadium II | 0 | 0 | 0 | 0 | 0 | 0 | 3 | 33 | 4 | 44 | 2 | 22 | 9 | 100 |
| Stadium III | 0 | 0 | 0 | 0 | 0 | 0 | 1 | 20 | 1 | 20 | 3 | 60 | 5 | 100 |
| Stadium IV | 1 | 6 | 1 | 6 | 0 | 0 | 5 | 29 | 5 | 29 | 5 | 29 | 17 | 100 |
| Verdacht auf Karzinom | 0 | 0 | 0 | 0 | 0 | 0 | 0 | 0 | 0 | 0 | 0 | 0 | 0 | 100 |
| ohne nähere Angabe | 1 | 6 | 0 | 0 | 0 | 0 | 9 | 56 | 4 | 25 | 2 | 13 | 16 | 100 |
| Insgesamt | 2 | 3,0 | 1 | 1,5 | 2 | 3,0 | 24 | 36,4 | 22 | 33,4 | 15 | 22,7 | 66 | 100,0 |

Tabelle V

Testergebnisse bei den Patientinnen mit Ovarialkarzinom nach Tumorstadien

| Tumorstadien | Testergebnisse 0 abs. | % | (−) abs. | % | (+) abs. | % | + abs. | % | ++ abs. | % | +++ abs. | % | Insgesamt abs. | % |
|---|---|---|---|---|---|---|---|---|---|---|---|---|---|---|
| Stadium I | 0 | 0 | 0 | 0 | 0 | 0 | 0 | 0 | 2 | 67 | 1 | 33 | 3 | 100 |
| Stadium II | 0 | 0 | 0 | 0 | 0 | 0 | 0 | 0 | 0 | 0 | 1 | 100 | 1 | 100 |

Tabelle V (Fortsetzung)

Testergebnisse bei den Patientinnen mit Ovarialkarzinom nach Tumorstadien

| Tumorstadien | Testergebnisse 0 abs. | % | (−) abs. | % | (+) abs. | % | + abs. | % | + + abs. | % | + + + abs. | % | Insgesamt abs. | % |
|---|---|---|---|---|---|---|---|---|---|---|---|---|---|---|
| Stadium III | 0 | 0 | 0 | 0 | 0 | 0 | 0 | 0 | 3 | 43 | 4 | 57 | 7 | 100 |
| Stadium IV | 0 | 0 | 2 | 5 | 3 | 7 | 4 | 10 | 14 | 34 | 18 | 44 | 41 | 100 |
| Verdacht auf Karzinom | 0 | 0 | 0 | 0 | 0 | 0 | 0 | 0 | 1 | 100 | 0 | 0 | 1 | 100 |
| ohne nähere Angabe | 1 | 6 | 0 | 0 | 1 | 6 | 3 | 17 | 8 | 44 | 5 | 28 | 18 | 100 |
| Insgesamt | 1 | 1,4 | 2 | 2,8 | 4 | 5,6 | 7 | 9,9 | 28 | 39,4 | 29 | 40,9 | 71 | 100 |

Tabelle VI

Testergebnisse bei den Patientinnen mit Korpuskarzinom nach Tumorstadien

| Tumorstadien | Testergebnisse 0 abs. | % | (−) abs. | % | (+) abs. | % | + abs. | % | + + abs. | % | + + + abs. | % | Insgesamt abs. | % |
|---|---|---|---|---|---|---|---|---|---|---|---|---|---|---|
| Stadium I | 3 | 13 | 2 | 8 | 0 | 0 | 2 | 8 | 9 | 38 | 8 | 33 | 24 | 100 |
| Stadium II | 0 | 0 | 1 | 8 | 0 | 0 | 3 | 23 | 5 | 38 | 4 | 31 | 13 | 100 |
| Stadium III | 0 | 0 | 0 | 0 | 0 | 0 | 1 | 20 | 1 | 20 | 3 | 60 | 5 | 100 |
| Stadium IV | 0 | 0 | 0 | 0 | 0 | 0 | 1 | 17 | 3 | 50 | 2 | 33 | 6 | 100 |
| Dysplasie | 0 | 0 | 1 | 100 | 0 | 0 | 0 | 0 | 0 | 0 | 0 | 0 | 1 | 100 |
| Verdacht auf Karzinom | 0 | 0 | 0 | 0 | 0 | 0 | 0 | 0 | 0 | 0 | 0 | 0 | 0 | 100 |
| ohne nähere Angabe | 0 | 0 | 0 | 0 | 2 | 9 | 5 | 22 | 13 | 57 | 3 | 13 | 23 | 100 |
| Insgesamt | 3 | 4,2 | 4 | 5,5 | 2 | 2,8 | 12 | 16,7 | 31 | 43,0 | 20 | 27,8 | 72 | 100,0 |

Tabelle VII

Testergebnisse bei den Patientinnen mit Kollumkarzinom nach Tumorstadien

| Tumorstadien | | 0 abs. | % | (−) abs. | % | (+) abs. | % | + abs. | % | + + abs. | % | + + + abs. | % | Insgesamt abs. | % |
|---|---|---|---|---|---|---|---|---|---|---|---|---|---|---|---|
| Stadium I | a | 1 | | 0 | | 2 | | 2 | | 6 | | 4 | | 15 | |
| | b | 0 | | 0 | | 0 | | 1 | | 0 | | 1 | | 2 | |
| | c | 0 | | 1 | | 1 | | 1 | | 3 | | 2 | | 8 | |
| | Zus. | 1 | 4 | 1 | 4 | 3 | 12 | 4 | 16 | 9 | 36 | 7 | 28 | 25 | 100 |
| Stadium II | a | 2 | | 1 | | 1 | | 0 | | 8 | | 10 | | 22 | |
| | b | 0 | | 0 | | 0 | | 0 | | 0 | | 1 | | 1 | |
| | c | 0 | | 0 | | 1 | | 0 | | 2 | | 11 | | 14 | |
| | Zus. | 2 | 5 | 1 | 3 | 2 | 5 | 0 | 0 | 10 | 27 | 22 | 60 | 37 | 100 |

Tabelle VII (Fortsetzung)

Testergebnisse bei den Patientinnen mit Kollumkarzinom nach Tumorstadien

| Tumorstadium | | 0 | | (−) | | (+) | | + | | ++ | | +++ | | Insgesamt | |
|---|---|---|---|---|---|---|---|---|---|---|---|---|---|---|---|
| | | abs. | % | abs. | % | abs. | % | abs. | % | abs. | % | abs. | % | abs. | % |
| Stadium III | a | 0 | | 0 | | 0 | | 2 | | 13 | | 21 | | 36 | |
| | b | 0 | | 0 | | 0 | | 0 | | 0 | | 0 | | 0 | |
| | c | 0 | | 0 | | 0 | | 1 | | 2 | | 13 | | 16 | |
| | Zus. | 0 | 0 | 0 | 0 | 0 | 0 | 3 | 6 | 15 | 29 | 34 | 65 | 52 | 100 |
| Stadium IV | a | 0 | | 0 | | 0 | | 2 | | 3 | | 6 | | 11 | |
| | b | 0 | | 0 | | 0 | | 0 | | 0 | | 0 | | 0 | |
| | c | 0 | | 0 | | 0 | | 2 | | 0 | | 2 | | 4 | |
| | Zus. | 0 | 0 | 0 | 0 | 0 | 0 | 4 | 27 | 3 | 20 | 8 | 53 | 15 | 100 |
| Dysplasie | a | 0 | | 0 | | 0 | | 0 | | 0 | | 0 | | 0 | |
| | b | 3 | | 2 | | 1 | | 2 | | 6 | | 2 | | 16 | |
| | c | 0 | | 0 | | 0 | | 0 | | 1 | | 1 | | 2 | |
| | Zus. | 3 | 17 | 2 | 11 | 1 | 6 | 2 | 11 | 7 | 39 | 3 | 17 | 18 | 100 |
| Carcinoma in situ | a | 1 | | 0 | | 0 | | 0 | | 0 | | 0 | | 1 | |
| | b | 1 | | 0 | | 0 | | 1 | | 3 | | 4 | | 9 | |
| | c | 1 | | 1 | | 0 | | 0 | | 2 | | 1 | | 5 | |
| | Zus. | 3 | 20 | 1 | 7 | 0 | 0 | 1 | 7 | 5 | 33 | 5 | 33 | 15 | 100 |
| Verdacht auf Karzinom | a | 0 | | 0 | | 0 | | 0 | | 0 | | 0 | | 0 | |
| | b | 0 | | 0 | | 0 | | 0 | | 0 | | 0 | | 0 | |
| | c | 0 | | 0 | | 0 | | 0 | | 0 | | 0 | | 0 | |
| | Zus. | 0 | 0 | 0 | 0 | 0 | 0 | 0 | 0 | 0 | 0 | 0 | 0 | 0 | 100 |
| ohne nähere Angabe | a | 0 | | 0 | | 0 | | 2 | | 6 | | 4 | | 12 | |
| | b | 0 | | 0 | | 0 | | 1 | | 1 | | 2 | | 4 | |
| | c | 1 | | 0 | | 0 | | 0 | | 2 | | 3 | | 6 | |
| | Zus. | 1 | 4 | 0 | 0 | 0 | 0 | 3 | 14 | 9 | 41 | 9 | 41 | 22 | 100 |
| Insgesamt | a | 4 | | 1 | | 3 | | 8 | | 36 | | 45 | | 97 | |
| | b | 4 | | 2 | | 1 | | 5 | | 10 | | 10 | | 32 | |
| | c | 2 | | 2 | | 2 | | 4 | | 12 | | 34 | | 56 | |
| | Zus. | 10 | 5,4 | 5 | 2,7 | 6 | 3,2 | 17 | 9,2 | 58 | 31,4 | 89 | 48,1 | 185 | 100,0 |

a = Kollumkarzinom; b = Portiokarzinom; c = Zervixkarzinom

Tabelle VIII

Testergebnisse bei den Patientinnen mit Vaginalkarzinom nach Tumorstadien

| Tumorstadium | Testergebnisse 0 | | (−) | | (+) | | + | | ++ | | +++ | | Insgesamt | |
|---|---|---|---|---|---|---|---|---|---|---|---|---|---|---|
| | abs. | % | abs. | % | abs. | % | abs. | % | abs. | % | abs. | % | abs. | % |
| Stadium I | 1 | 50 | 0 | 0 | 0 | 0 | 0 | 0 | 1 | 50 | 0 | 0 | 2 | 100 |
| Stadium II | 0 | 0 | 0 | 0 | 0 | 0 | 0 | 0 | 0 | 0 | 1 | 100 | 1 | 100 |
| Stadium III | 0 | . | 0 | . | 0 | . | 0 | . | 0 | . | 0 | . | 0 | . |
| Stadium IV | 0 | 0 | 0 | 0 | 0 | 0 | 0 | 0 | 1 | 50 | 1 | 50 | 2 | 100 |
| Dysplasie | 0 | . | 0 | . | 0 | . | 0 | . | 0 | . | 0 | . | 0 | . |
| Carcinoma in situ | 0 | 0 | 0 | 0 | 0 | 0 | 0 | 0 | 0 | 0 | 1 | 100 | 1 | 100 |

Tabelle VIII (Fortsetzung)

Testergebnisse bei den Patientinnen mit Vaginalkarzinom nach Tumorstadien

| Tumorstadium | Testergebnisse 0 abs. | % | (−) abs. | % | (+) abs. | % | + abs. | % | + + abs. | % | + + + abs. | % | Insgesamt abs. | % |
|---|---|---|---|---|---|---|---|---|---|---|---|---|---|---|
| Verdacht auf Karzinom | 0 | . | 0 | . | 0 | . | 0 | . | 0 | . | 0 | . | 0 | . |
| ohne nähere Angabe | 0 | 0 | 0 | 0 | 0 | 0 | 2 | 14 | 6 | 43 | 6 | 43 | 14 | 100 |
| Insgesamt | 1 | 5 | 0 | 0 | 0 | 0 | 2 | 10 | 8 | 40 | 9 | 45 | 20 | 100 |

Tabelle IX

Testergebnisse bei den Patientinnen mit Vulvakarzinom nach Tumorstadien

| Tumorstadien | Testergebnisse 0 abs. | % | (−) abs. | % | (+) abs. | % | + abs. | % | + + abs. | % | + + + abs. | % | Insgesamt abs. | % |
|---|---|---|---|---|---|---|---|---|---|---|---|---|---|---|
| Stadium I | 0 | . | 0 | . | 0 | . | 0 | . | 0 | . | 0 | . | 0 | . |
| Stadium II | 0 | 0 | 0 | 0 | 1 | 33 | 1 | 33 | 1 | 33 | 0 | 0 | 3 | |
| Stadium III | 0 | 0 | 0 | 0 | 0 | 0 | 0 | 0 | 0 | 0 | 2 | 100 | 2 | |
| Stadium IV | 0 | 0 | 1 | 33 | 0 | 0 | 0 | 0 | 1 | 33 | 1 | 33 | 3 | |
| Dysplasie | 0 | . | 0 | . | 0 | . | 0 | . | 0 | . | 0 | . | 0 | . |
| Carcinoma in situ | 0 | . | 0 | . | 0 | . | 0 | . | 0 | . | 0 | . | 0 | . |
| Verdacht auf Karzinom | 0 | . | 0 | . | 0 | . | 0 | . | 0 | . | 0 | . | 0 | . |
| ohne nähere Angabe | 0 | 0 | 0 | 0 | 0 | 0 | 2 | 50 | 0 | 0 | 2 | 50 | 4 | |
| Insgesamt | 0 | 0 | 1 | 8 | 1 | 8 | 3 | 25 | 2 | 17 | 5 | 42 | 12 | 100 |

Tabelle X

Testergebnisse bei den Patientinnen, die den angegebenen Diagnosegruppen (ausser Karzinomen im Bereich der Genitalorgane) angehören

| Diagnosen-gruppe | Testergebnisse 0 abs. | % | (−) abs. | % | (+) abs. | % | + abs. | % | + + abs. | % | + + + abs. | % | Insgesamt abs. | % |
|---|---|---|---|---|---|---|---|---|---|---|---|---|---|---|
| Malignome (ausser denen im Genitalbereich) | 1 | 6 | 0 | 0 | 0 | 0 | 1 | 6 | 7 | 38 | 9 | 50 | 18 | 100 |
| Gutartige Tumoren | 2 | 9 | 3 | 13 | 5 | 23 | 2 | 9 | 5 | 23 | 5 | 23 | 22 | 100 |
| Entzündungen (vorwiegend Genitalbereich) | 4 | 34 | 1 | 8 | 1 | 8 | 3 | 25 | 2 | 17 | 1 | 8 | 12 | 100 |

Tabelle X (Fortsetzung)

Testergebnisse bei den Patientinnen, die den angegebenen Diagnosegruppen (ausser Karzinomen im Bereich der Genitalorgane) angehören

| | Testergebnisse | | | | | | | | | | | | | |
|---|---|---|---|---|---|---|---|---|---|---|---|---|---|---|
| Diagnosen- | 0 | | (−) | | (+) | | + | | ++ | | +++ | | Insgesamt | |
| gruppe | abs. | % | abs. | % | abs. | % | abs. | % | abs. | % | abs. | % | abs. | % |
| Diagnostische Restgruppe (z.Teil ohne Befund) | 1 | 13 | 0 | 0 | 2 | 25 | 1 | 13 | 3 | 37 | 1 | 13 | 8 | 100 |

In der nachfolgenden Tabelle XI sind die Testergebnisse zusammengefasst, die bei Karzinomen gefunden worden sind.

Tabelle XI

Testergebnisse bei den Patientinnen mit Mammakarzinom, Ovarialkarzinom, Kollumkarzinom und Vulvakarzinom (nur Fälle mit der Kennzeichnung des Tumorstadiums I, II, III, IV wurden berücksichtigt)

| Testergebnise | | | | | | | | | | | | | |
|---|---|---|---|---|---|---|---|---|---|---|---|---|---|
| 0 | | (−) | | (+) | | + | | ++ | | +++ | | Insgesamt | |
| abs. | % | abs. | % | abs. | % | abs. | % | abs. | % | abs. | % | abs. | % |
| 8 | 2,7 | 9 | 3,1 | 11 | 3,8 | 38 | 13,0 | 96 | 32,9 | 130 | 44,5 | 292 | 100,0 |

Daraus ist ersichtlich, dass die diagnostische Vorrichtung eine bisher unerreichbare hohe Treffsicherheit aufweist und für eine Differentialdiagnostik geeignet ist. Bei der statistischen Auswertung der Testergebnisse zeigte sich, dass in allen Fällen, in denen durch andere Untersuchungsmethoden das Vorliegen von Karzinomen (Stadium I bis IV) im Genitalbereich gesichert war (292 Fälle), die diagnostische Vorrichtung nur zu 9,6% falsch-negativen Diagnosen führte.

**Patentansprüche**

1. Diagnostische Vorrichtung zum Nachweis einer erhöhten Konzentration von Dehydrogenasen und/oder Oxidasen in Flüssigkeiten von Menschen, Tieren oder Pflanzen, bestehend aus einem Träger, der einen Redox-Farbstoff bindet, sowie einem auf einen pH-Wert im sauren Bereich eingestellten Stoffgemisch aus dem der jeweiligen Dehydrogenase entsprechenden Substrat, einer wasserstoffübertragenden Verbindung und mindestens einem Redox-Farbstoff, dadurch gekennzeichnet, dass ein aus Cellulosefasern gebildeter polare Gruppen aufweisender Träger verwendet wird und das Stoffgemisch zur Erfassung von ausschliesslich pathologisch erhöhten Konzentrationen der jeweiligen Dehydrogenase und/oder Oxidase auf einen pH-Wert von unter 5,0 eingestellt ist.

2. Diagnostische Vorrichtung nach Anspruch 1, dadurch gekennzeichnet, dass der pH-Wert auf mindestens 3,0 eingestellt ist.

3. Diagnostische Vorrichtung nach Anspruch 1 oder 2, dadurch gekennzeichnet, dass das Stoffgemisch zur Einstellung und/oder Aufrechterhaltung des pH-Wertes ein Puffersystem enthält.

4. Diagnostische Vorrichtung nach Anspruch 3, dadurch gekennzeichnet, dass das Puffersystem in Form von Triäthanolaminhydrochlorid oder in Form eines nicht äquivalenten Gemisches aus Triäthanolamin und Chlorwasserstoff vorliegt.

5. Diagnostische Vorrichtung nach einem der vorstehenden Ansprüche, dadurch gekennzeichnet, dass das Stoffgemisch zusätzlich eine Verbindung enthält, die als Wasserstoffüberträger oder Elektronenüberträger wirkt.

6. Diagnostische Vorrichtung nach einem der vorstehenden Ansprüche, dadurch gekennzeichnet, dass der Träger aus einem Tampon besteht, der Cellulosefasern enthält.

7. Verwendung der diagnostischen Vorrichtung nach einem der Ansprüche 1 bis 6 zum intra- oder extrakorporalen Nachweis einer pathologisch erhöhten Konzentration von Dehydrogenasen in menschlichen, tierischen oder pflanzlichen extrazellulären Flüssigkeiten, wobei das Stoffgemisch auf dem Träger mit der zu untersuchenden Flüssigkeit umgesetzt wird, dadurch gekennzeichnet, dass die Umsetzung des Stoffgemisches mit der zu untersuchenden Flüssigkeit während höchstens 10 Minuten durchgeführt wird.

8. Verwendung der diagnostischen Vorrichtung nach einem der Ansprüche 1 bis 6, dadurch gekennzeichnet, dass die Umsetzung während 5 bis 7 Minuten durchgeführt wird.

**Claims**

1. Diagnostic device for the detection of an increased concentration of dehydrogenases and/or oxidases in the fluids of humans, animals or plants, which consists of a carrier which bonds a redox dyestuff, and a substance mixture, adjusted to a pH value in the acid range, of the substrate corresponding to the particular dehydrogenase, a hydrogen donor compound and at least one redox dyestuff, characterised in that use is made of a carrier which has polar groups and is formed of cellulose fibres, and the substance mixture for detecting exclusively pathologically increased concentrations of the particular dehydrogenase and/or oxidase is adjusted to a pH value of below 5.0.

2. Diagnostic device according to Claim 1, characterised in that the pH value is adjusted to at least 3.0.

3. Diagnostic device according to Claim 1 or 2, characterised in that the substance mixture contains a buffer system for establishing and/or maintaining the pH value.

4. Diagnostic device according to Claim 3, characterised in that the buffer system in the form of triethanolamine hydrochloride or in the form of a non-equivalent mixture of triethanolamine and hydrogen chloride.

5. Diagnostic device according to one of the preceding claims, characterised in that the substance mixture additionally contains a compound which acts as a hydrogen donor or electron donor.

6. Diagnostic device according to one of the preceding claims, characterised in that the carrier consists of a tampon containing cellulose fibres.

7. Use of the diagnostic device according to one of Claims 1 to 6 for itracorporal or extracorporal detection of a pathologically increased concentration of dehydrogenases in extracellular human, animal or vegetable fluids, the substance mixture on the carrier being reacted with the fluid to be investigated, characterised in that the reaction of the substance mixture with the fluid to be investigated is carried out for at most 10 minutes.

8. Use of the diagnostic device according to one of Claims 1 to 6, characterised in that the reaction is carried out for 5 to 7 minutes.

**Revendications**

1. Dispositif pour diagnostic destiné à mettre en évidence une concentration accrue en déhydrogénases et/ou oxydases dans les liquides d'êtres humains, d'animaux ou de plantes, constitué par un support qui lie un colorant Redox ainsi que par un mélange de matières ajusté à une valeur de pH dans le domaine acide constitué à partir du substrat correspondant à la déhydrogénase considérée, par un composé transférant l'hydrogène et par au moins un colorant Redox, caractérisé en ce que l'on utilise un support comportant des groupes polaires et formé à partir de fibres de cellulose et en ce que le mélange de matières est ajusté à une valeur de pH inférieure à 5,0 pour la détection exclusive de concentrations pathologiquement accrues de la déhydrogénase et/ou de l'oxydase considérée.

2. Dispositif pour diagnostic selon la revendication 1, caractérisé en ce que la valeur du pH est ajustée à au moins 3,0.

3. Dispositif pour diagnostic selon la revendication 1 ou 2, caractérisé en ce que le mélange de matières contient un système tampon pour l'ajustement et/ou le maintien de la valeur du pH.

4. Dispositif pour diagnostic selon la revendication 3, caractérisé en ce que le système tampon se présente sous forme de chlorhydrate de triéthanolamine ou sous forme d'un mélange non équivalent constitué de triéthanolamine et de gaz chlorhydrique.

5. Dispositif pour diagnostic selon l'une des revendications précédentes, caractérisé en ce que le mélange de matières contient de plus un composé qui agit comme agent de transfert d'hydrogène ou agent de transfert d'électrons.

6. Dispositif pour diagnostic selon l'une des revendications précédentes, caractérisé en ce que le support consiste en un tampon qui contient des fibres de cellulose.

7. Utilisation du dispositif pour diagnostic selon l'une des revendications 1 à 6 pour la mise en évidence intra- ou extracorporelle d'une concentration accrue en déhydrogénases pathologiquement dans les liquides extracellulaires d'êtres humains, d'animaux ou de végétaux, le mélange de matières étant mis en réaction sur le support avec le liquide à examiner, caractérisée en ce que la réaction du mélange de matières avec le liquide à examiner est effectuée pendant au maximum 10 minutes.

8. Utilisation du dispositif pour diagnostic selon l'une des revendications 1 à 6, caractérisée en ce que la réaction est effectuée en 5 à 7 minutes.